# EUROPEAN PATENT SPECIFICATION

(11) **EP 3 285 967 B1**
(45) Date of publication and mention of the grant of the patent: **06.03.2019**
(21) Application number: 16721237.2
(22) Date of filing: 12.04.2016
(51) Int. Cl.: B25D 17/00, F16P 3/14, G07C 9/00

(54) **APPARATUS AND METHOD FOR SAFEGUARDING USERS DURING USE OF APPARATUS HAVING AT LEAST A PART IN MOTION**
VORRICHTUNG UND VERFAHREN ZUM SCHUTZ VON BENUTZERN WÄHREND DER VERWENDUNG EINER VORRICHTUNG MIT MINDESTENS EINEM TEIL IN BEWEGUNG
APPAREIL ET PROCÉDÉ POUR PROTÉGER DES UTILISATEURS PENDANT L'UTILISATION D'UN APPAREIL AYANT AU MOINS UNE PARTIE EN MOUVEMENT

(30) Priority: 20.04.2015 IT VR20150063
(43) Date of publication of application: 28.02.2018
(73) Proprietor: Future Smart S.r.l., 37059 Zevio (Verona) (IT)
(72) Inventor: MARCATO, Maurizio, 37030 Verona (IT)
(74) Representative: Anselmi, Davide
(86) International application number: PCT/IB2016/052065
(87) International publication number: WO 2016/170448

(56) References cited:
- GB-A- 2 473 061
- US-A- 5 563 581
- US-A1- 2012 319 846

## Description

This invention relates to an apparatus and a method for safeguarding users during use of apparatus having at least a part in motion. More specifically, this invention belongs to the field of safety at work or at home in relation to the use of tools which involve use by the users. Examples of these electrical tools are: drills, electric saws, nail guns, ....

Preferably, this invention relates to the sector of electrical tools wherein the motion is powered by electricity. However, this invention may also be used for apparatuses wherein the moving part is moved by means of a combustion engine (for example a chain saw) or by other means not expressly mentioned herein.

Reference is made hereinafter mainly to tools of the electric type, without thereby excluding the possibility of tools powered by a combustion engine or other means.

In effect, according to the prior art, during use of electrical tools the users can injure themselves due to the movement of the tool itself. For example, if the tool is a drill, the user's hand can remain trapped at the drill bit of the drill due, for example, to wrapping of the glove, and then twisting on itself, causing fracturing of the hand, wounds, amputations, etc.

More specifically, during the use of a tool, the user can be injured due to the fact that the movement of the tool continues even when the user has already found him/herself in a dangerous condition. However, the user is so concentrated on the area for attaching/fixing to the part of the body that instead of disabling the tool (for example by releasing the pushbutton of the drill) the user tries to free the part of the body in danger (thereby increasing even further the risk of injury).

Some examples of devices for safeguarding users are described in patent document US 2012/0319846 and involve the use of a proximity sensor (to be applied to the user) to check the distance of the user from the apparatus in such a way as to switch the apparatus ON/OFF only if the user is in the correct position.

In this situation, the aim of this invention is to provide an apparatus and a method for safeguarding users during the use of apparatuses with at least a part in motion which overcomes the above-mentioned drawbacks.

More specifically, the aim of this invention is to provide an apparatus and a method for safeguarding users during the use of apparatuses with at least a part in motion which allows the physical damage caused by these apparatuses to be reduced in the case of danger.

Another aim of this invention is to provide an apparatus and a method for safeguarding users during the use of apparatuses with at least a part in motion which allows the management of the electrical apparatus to be optimised in the case of danger.

The aims indicated are substantially achieved by an apparatus and a method for safeguarding users during the use of apparatuses with at least a part in motion as described in the appended claims.

Further characteristic features and advantages of this invention will emerge more clearly from the detailed description of several preferred, but not exclusive embodiments of an apparatus and a method for safeguarding users during use of apparatus having at least a part in motion illustrated in the accompanying drawings, in which:
- Figure 1 is a schematic view of a first preferred embodiment of the apparatus according to this invention;
- Figure 2 is a schematic view of a second preferred embodiment of the apparatus according to this invention; and
- Figure 3 is a schematic view of a third preferred embodiment of the apparatus according to this invention.

With reference to the above-mentioned drawings, the numeral 1 denotes in its entirety an apparatus 1 for safeguarding users during use of apparatuses 2 with at least a part in motion according to this invention. More specifically, the apparatus 1 comprises a management device 3 of the part in motion in use connected to the part in motion of the apparatus 2 to the apparatus 2. More specifically, the management device 3 is configurable between an enabled condition during which the motion of the part of the apparatus 2 is enabled, and an interruption condition during which the motion of the part of the apparatus 2 is disabled. In detail, the management device 3 may be connected to a system for controlling the moving part of the apparatus 2 irrespective of the type of movement means connected to the part in motion. In other words, the movement means may be defined by an electric motor, an actuator, a switch, a combustion engine or other...

For example, in the case of an apparatus 2 powered by a combustion engine (for example, a chain saw) the management device 3 is configured to act on the combustion engine (for example by means of an actuator) in order to manage the movement.

In any case, the management device 3 can be connected to a panic element (for example, a button) which is often connected to the industrial apparatuses 2 for stopping them if they are deactivated. In effect, in this case, the panic element already comprises a system for controlling the apparatus 2.

In any case, the management device 3 may act directly on an electrical control circuit of the apparatus 2 by means of electrical signals or may act mechanically on the part in motion by means of a suitable actuator.

In the case shown in the accompanying drawings, the management device 3 is interposed and connected between a source 4 of electric current and an electrical apparatus 2. The accompanying drawings show the source 4 of electricity (by way of example represented by an electrical socket) and an electrical apparatus 2 (drill).

In that case, the flow of electric current from the source 4 of electric current towards the apparatus 2 is enabled during the enabling condition, whilst there is no flow of electric current from the source 4 of electric current towards the apparatus 2 during the interruption condition.

It should be noted that the management device 3 is connected to the electric apparatus 2 by an electric cable 5. Thus, the management device 3 is interposed between the electricity socket and the electric cable 5 which leads to the electrical apparatus 2.

Preferably, the management device 3 comprises an electrical plug 6 which can be inserted in the electrical socket of the source 4 of electricity.

In other words, the management device 3 comprises an input for the electric current coming from the energy source and an output to which the electric apparatus 2 is connected.

In detail, the management device 3 comprises means for interrupting the current interposed between the input and the output in such a way as to interrupt the current during the interruption condition or allow the flow of current during the flow condition. It should be noted that the management device 3 may be implemented by any technically equivalent electrical device.

Moreover, the apparatus 1 comprises a measuring device 7 of at least a biometric value associated in use to the user's body for measuring the biometric value. More specifically, the measuring device 7 may be external such as, for example, a bracelet, a necklace, a belt, a chest strap, ... or internal such as, for example, a chip or a detector under the skin.

In the preferred embodiment illustrated in the accompanying drawings, the measuring device 7 of the biometric value comprises a bracelet wearable on a user's arm and a sensor 14 for measuring the biometric value by contact with the user's body. Preferably, the sensor 14 for measuring the biometric value is a sensor for measuring a heartbeat. However, in other embodiments not expressly described here, the sensor 14 could measure other biometric values (adrenaline in the blood, ....).

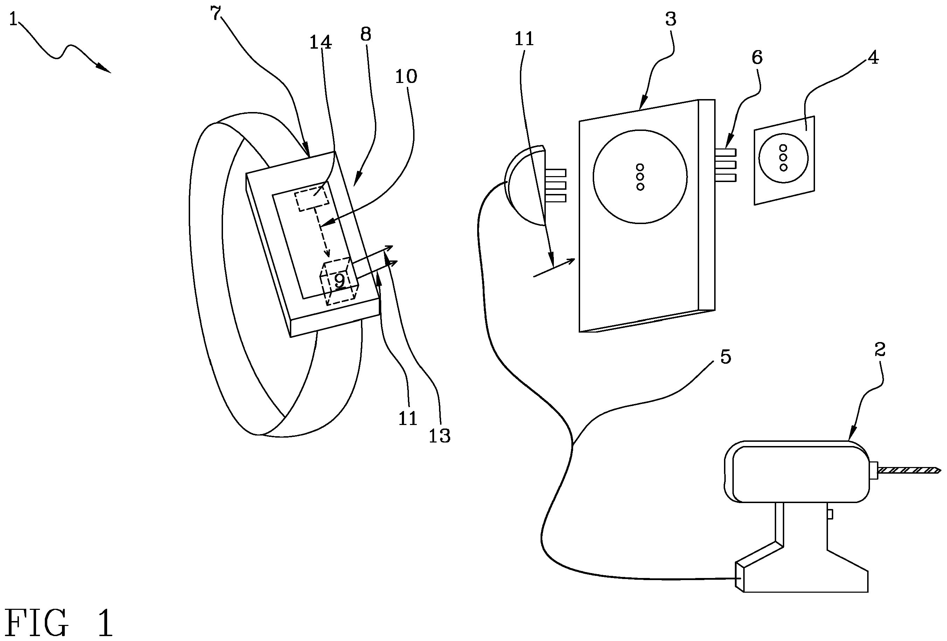

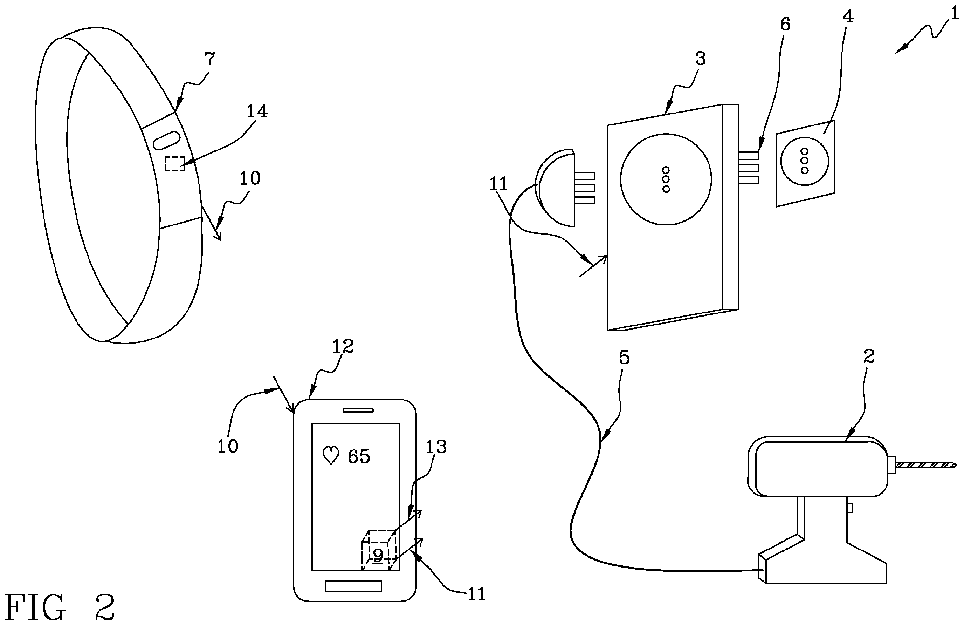

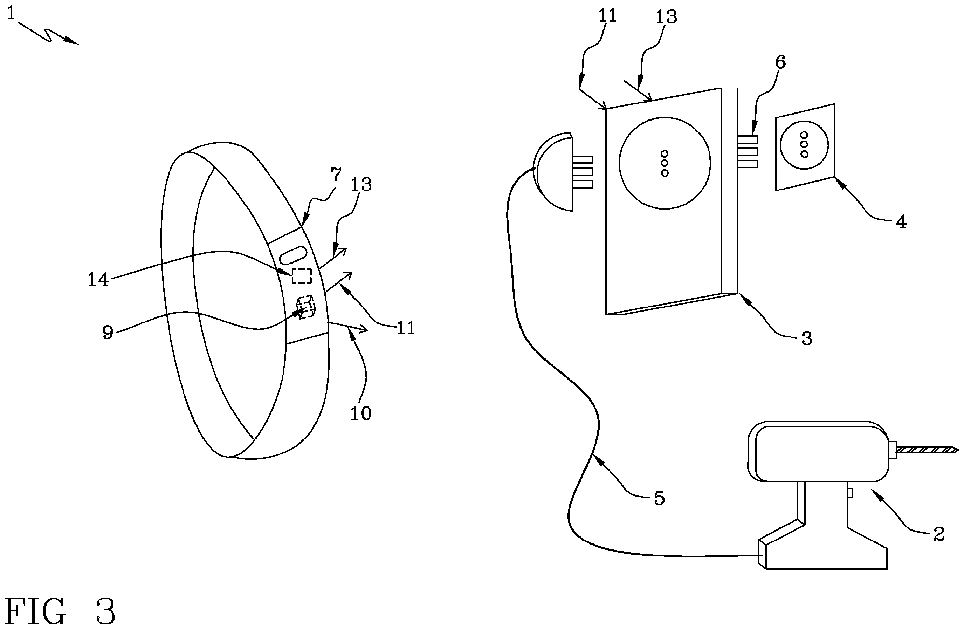

More specifically, the device for measuring the biometric value may be a bracelet specially configured to measure a particular biometric value (Figures 2 and 3) or it may be integrated in a predetermined and already existing bracelet such as, for example, a watch. In detail, the bracelet may be a smartwatch 8 (preferably already configured for measuring the heartbeat) as illustrated in Figure 1.

In accordance with this invention, the apparatus 1 comprises a processing unit 9 operatively connected to the management device 3 of the electric current and to the measuring device 7.

The processing unit 9 is configured for receiving a measuring signal 10 of the biometric value containing the biometric value measured by the measuring device 7. In other words, the measuring device 7 is configured to send the measuring signal 10 to the processing unit 9 where the measuring signal 10 contains the data relative to the measured biometric value.

Moreover, the processing unit 9 is configured for measuring a sudden variation in the measured biometric value relative to a reference biometric value. This biometric reference value may be calculated in an objective or subjective manner.

In any case, the reference biometric value indicates the presence of the user in a normal or customary or rest condition of life (from a medical point of view).

In other words, the processing unit 9 is configured to receive a signal containing the biometric reference value. The latter can be calculated by the apparatus 1 itself, or pre-memorised in an internal memory or the signal can arrive directly from the outside.

Preferably, the reference biometric value corresponds to one of the last measured biometric values or it is the average of the last measured biometric values. The term "last" is used to mean the biometric values measured most recently relative to the sudden variation of the reference biometric value.

In other words, the processing unit 9 is configured for comparing the measured biometric value with the reference biometric value. The latter may correspond to the most recent biometric values or may correspond to an independent predetermined biometric value.

Moreover, the processing unit 9 is configured for sending a disabling signal 11 to the management device 3 so as to configure the management device 3 from the enabled condition to the interruption condition when the biometric value distances from the reference biometric value by a reference differential value so as to block the motion of the part in motion of the apparatus 2. Obviously, the management device 3 is configured for receiving the disabling signal 11 and for configuring the latter from the enabling condition to the interruption condition.

More specifically, the processing unit 9 is configured for sending the disabling signal 11 to the management device 3 so as to configure the management device from the enabled condition to the interruption condition when the biometric value measured reaches or exceeds an altered biometric value representing a condition of presence of the user in a condition of altered life so as to disable the motion of the part of the apparatus 2. This altered biometric value is different from the reference biometric value by a differential reference value. Preferably, the altered biometric value is greater than the reference biometric value by the differential reference value.

In other words, in the case of sudden variation of the biometric value, the processing unit 9 is configured for disabling the movement of the part of the apparatus 2 to safeguard the user. The expression "sudden variation" means a variation of the biometric value over time which is faster than the variations, over time, the biometric value during the normal or customary life condition of the user.

In the preferred case, the measured biometric value is the heartbeat and the reference biometric value is defined by a heartbeat value in a condition of normal life of the user (e.g. number of heartbeats per minute), whilst the altered biometric value is defined by a heartbeat value greater than the heartbeat value of the normal life condition. In other words, when the value of heartbeats increases above a certain threshold, the processing unit 9 is configured to disable the movement of the part of the apparatus 2.

As already mentioned, if the movement is generated by movement means of the electric type the disabling signal 11 is used to disable the movement means or to interrupt the power supply signal. If the movement is generated by movement means of the combustion or other type, the disabling signal is used to act on the control system of the movement means.

In the preferred embodiment illustrated in the accompanying drawings, the processing unit 9 is configured for sending the disabling signal 11 to the management device 3 in order to configure the latter from the enabling condition to the interruption condition in such a way as to disconnect the apparatus 2 from the electric current. Obviously, the management device 3 is configured for receiving the disabling signal 11 and for configuring the latter from the enabling condition to the interruption condition.

In other words, in the case of sudden variation of the biometric value, the processing unit 9 is configured to disable the flow of electric current from the source 4 of electricity to the electric apparatus 2 in such a way as to interrupt the power supply of the latter so as to safeguard the user. Advantageously, the invention enables the movement of the part in motion to be blocked when a sudden variation of the biometric value in question is detected (for example, an increase in the heart rate). In that way, it is possible to safeguard the user by interrupting the movement of the part of the electric apparatus 2 which the user is using.

It should be noted that the reference differential value is a function of the variation which the measured biometric must have to define a change such as to correspond to the occurrence of an event which is dangerous for the user.

In an embodiment of the invention illustrated in Figure 3, the processing unit 9 is housed on the management device 3. In this way, the measuring device 7 (bracelet) measures the biometric value and transmits the measuring signal 10 (preferably via wireless) to the management device 3. In detail, the measuring signal 10 is received by the processing unit 9 which interrupts the movement (for example interrupting the supply of electric current). Even more in detail, the disabling signal 11 is transmitted inside the measuring device 7 between the processing unit 9 and the management device 3.

In an alternative embodiment, the processing unit 9 is housed on the measuring device 7 of the biometric value. In this way, the measuring signal 10 is transmitted inside the measuring device 7 towards the processing unit 9, whilst the disabling signal 11 is transmitted (preferably via wireless) by the processing unit 9 towards the management device 3. Figure 3 illustrates a measuring device 7 integrated in a smartwatch 8 which is already configured to measure the biometric value (heartbeat) and is further configured for processing the measured biometric value as a function of a reference value and for sending the disabling signal 11 to the management device 3.

In a further embodiment illustrated in Figure 2, the apparatus 1 comprises a smartphone 12 and/or a tablet PC and/or computer of known type comprising a relative processing unit. The processing unit 9 is defined by the processing card of the smartphone 12 and/or tablet and/or computer. In other words, the processing unit of the smartphone 12 and/or a tablet and/or computer is operatively connected to the measuring device 7 and to the management device 3 and is configured for:
- receiving the measuring signal 10 of the biometric value containing the biometric value measured by the measuring device 7;
- comparing the biometric value measured with the reference biometric value wherein the biometric reference value is represented by a condition of presence of the user in a condition of usual life;
- sending the disabling signal 11 to the management device 3 so as to configure the management device from the enabled condition to the interruption condition when the biometric value measured reaches or exceeds the altered biometric value so as to disable the motion of the part of the apparatus 2.

It should be noted that the disabling signal 11 is transmitted from the smartphone 12 and/or tablet and/or computer to the management device 3 of the current via wireless. In the same way, the measuring signal 10 is transmitted by the measuring device 7 to the smartphone 12 and/or tablet and/or computer via wireless.

Lastly, it should be noted that the control unit is further configured for sending a re-enabling signal 13 to the management device 3 when the biometric value moves towards the reference biometric value relative to the differential value configuring the management device 3 from the interruption condition to the enabling condition.

This invention also relates to a method for safeguarding users during use of apparatuses 2 having at least a part in motion. The method is derived directly from what is described above with regard to the apparatus 1, which is here below incorporated in its entirety. More specifically, the method comprises a first step of connecting the management device 3 of the movement of the part of the apparatus 2 to the apparatus 2, wherein the management device 3 is configurable between the enabling condition and the interruption condition during which the movement of the part of the apparatus 2 is not is enabled.

Moreover, the method comprises a second step of measuring the biometric value of the user's body. Preferably, the second step is carried out by a sensor/detector 14 of the biometric value as described above.

In addition, the method comprises the step of comparing the measured biometric value with the reference biometric value in such a way as to check if there is a predetermined variation of the measured biometric value. In other words, it is detected if the measured biometric value moves away from the reference biometric value by the reference differential value in such a way as to disconnect the apparatus 2 from the electric current.

In that case, a disabling signal 11 is sent to the management device 3 so as to configure the management device 3 from the enabled condition to the interruption condition.

The invention achieves the preset aims.

More specifically, the invention makes it possible to safeguard users during the use of tools by interrupting the movement in the case of danger. In effect, in these dangerous situations the measuring device 7 detects a variation of a biometric parameter (e.g. heartbeat) of the user. The variation of the biometric parameter is the direct consequence of the user finding him/herself in a dangerous situation. Consequently, to avoid increasing the damage, the apparatus 1 according to this invention immediately switches OFF the current or disables the movement in another manner so as to disable the electrical apparatus 2.

In that way, the health of the users is safeguarded in a simple and optimum manner.

It should also be noted that this invention is relatively easy to implement and that the cost of implementing the invention is relatively low.

## Claims

1. An apparatus (1) for safeguarding users during use of apparatus (2) having at least a part in motion, comprising:
- a management device (3) of a motion of the part of the apparatus (2) in use connected to the apparatus (2); said management device (3) being configurable between an enabled condition during which the motion of the part of the apparatus (2) is enabled, and an interrupted condition during which the motion of the part of the apparatus (2) is disabled;
- a measuring device (7) of at least a biometric value associated in use to the user's body for measuring the biometric value;
**characterised in that** it comprises a processing unit (9) operatively connected to the management device (3) of the motion and to the measuring device (7); the processing unit (9) being configured for:
- receiving a measuring signal (10) of the biometric value containing the biometric value measured by the measuring device (7);
- comparing the biometric value measured with a reference biometric value wherein the biometric reference value is represented by a condition of presence of the user in a condition of usual life;
- sending a disabling signal (11) to the management device (3) of the motion so as to configure the management device from the enabled condition to the interruption condition when the biometric value measured and contained in the measuring signal (10) reaches or exceeds an altered biometric value representing a condition of presence of the user in a condition of altered life so as to disable the motion of the part of the apparatus (2); the altered biometric value being different from the reference biometric value.

2. The apparatus (1) according to claim 1, **characterised in that** the processing unit (9) is configured to disable the movement of the part of the apparatus (2) when it detects a sudden variation of the measured biometric value from the reference biometric value to the altered biometric value.

3. The apparatus (1) according to any one of the preceding claims, **characterised in that** the altered biometric value is greater than the measured biometric value.

4. The apparatus (1) according to any one of the preceding claims, **characterised in that** the management system (3) is connected in use to a control system of the part in motion for managing the part in motion.

5. The apparatus (1) according to any one of claims 1 to 3, **characterised in that** the management system (3) is in use interposed between and connected to a source (4) of electrical energy and the electrical supply of the apparatus (2).

6. The apparatus (1) according to any one of the preceding claims, **characterised in that** the reference biometric value is substantially equal to the most recently measured biometric value.

7. The apparatus (1) according to any one of claims 1 to 5, **characterised in that** the reference biometric value is substantially equal to the average of the most recently measured biometric values.

8. The apparatus (1) according to any one of the preceding claims, **characterised in that** the processing unit (9) is housed on the management device (3).

9. The apparatus (1) according to any one of claims 1 to 7, **characterised in that** the processing unit (9) is housed on the measuring device (7) of the biometric value.

10. The apparatus (1) according to any one of claims 1 to 7, **characterised in that** it comprises a smartphone (12) and/or a tablet and/or a computer comprising its own processing card; the processing unit (9) being defined by the processing card of the smartphone (12) and/or tablet and/or computer.

11. The apparatus (1) according to any one of claims 1 to 7, **characterised in that** the measuring device (7) of the biometric value comprises a bracelet wearable on a user's arm and a sensor (14) for measuring the biometric value by contact with the user's body.

12. The apparatus (1) according to any one of the preceding claims, **characterised in that** the device (7) for measuring the biometric value comprises a sensor (14) for measuring the heartbeat in such a way that the heartbeat defines the biometric value; the reference biometric value being defined by a heartbeat value in a condition of normal life of the user, the altered biometric value being defined by a heartbeat value greater than the heartbeat value of the normal life condition.

13. The apparatus (1) according to any one of the preceding claims, **characterised in that** the disabling signal (11) is transmitted by the processing unit (9) to the management device (3) via wireless.

14. The apparatus (1) according to any one of the preceding claims, **characterised in that** the measuring signal (10) is transmitted by the processing unit (7) to the processing device (9) via wireless.

15. A method for safeguarding users during use of apparatus (2) having at least a part in motion, comprising following operating steps:
- connecting a management device (3) of the part in motion of the apparatus (2) to the apparatus (2), wherein the management device (3) is configurable between an enabled condition during which the motion of the part of the apparatus (2) is enabled, and an interruption condition during which the motion of the part of the apparatus (2) is disabled;
- measuring a biometric value of the user's body;
**characterised in that** it comprises following operating steps of:
comparing the biometric value measured with a reference biometric value wherein the biometric reference value is represented by a condition of presence of the user in a condition of usual life;
- sending a disabling signal (11) to the management device (3) so as to configure the management device from the enabled condition to the interruption condition when the biometric value measured reaches or exceeds an altered biometric value representing a condition of presence of the user in a condition of altered life so as to disable the motion of the part of the apparatus (2); the altered biometric value being different from the reference biometric value.

## Patentansprüche

1. Vorrichtung (1) zum Schutz von Benutzern während der Verwendung einer Vorrichtung (2) mit mindestens einem Teil in Bewegung, umfassend:
- ein Verwaltungsgerät (3) einer Bewegung des Teils der Vorrichtung (2) bei Verwendung, das mit der Vorrichtung (2) verbunden ist, wobei das Verwaltungsgerät (3) zwischen einem aktivierten Zustand, in dem die Bewegung des Teils der Vorrichtung (2) aktiviert ist, und einem unterbrochenen Zustand, in dem die Bewegung des Teils der Vorrichtung (2) deaktiviert ist, konfigurierbar ist;
- ein Messgerät (7) von mindestens einem biometrischen Wert, das bei Verwendung mit dem Körper des Benutzers zur Messung des biometrischen Werts assoziiert ist; **dadurch gekennzeichnet, dass** sie eine Verarbeitungseinheit (9) umfasst, die operativ mit dem Verwaltungsgerät (3) der Bewegung und mit dem Messgerät (7) verbunden ist, wobei die Verarbeitungseinheit (9) konfiguriert ist zum:
- Empfangen eines Messsignals (10) des biometrischen Werts, der den vom Messgerät (7) gemessenen biometrischen Wert enthält;
- Vergleichen des gemessenen biometrischen Werts mit einem biometrischen Referenzwert, wobei der biometrische Referenzwert durch einen Anwesenheitszustand des Benutzers in einem normalen Lebenszustand dargestellt wird;
- Senden eines Deaktivierungssignals (11) an das Verwaltungsgerät (3) der Bewegung, um das Verwaltungsgerät von dem aktivierten Zustand in den Unterbrechungszustand zu konfigurieren, wenn der im Messsignal (10) gemessene und enthaltene biometrische Wert einen veränderten biometrischen Wert erreicht oder überschreitet, der einen Anwesenheitszustand des Benutzers in einem veränderten Lebenszustand darstellt, um die Bewegung des Teils der Vorrichtung (2) zu deaktivieren, wobei der veränderte biometrische Wert sich vom biometrischen Referenzwert unterscheidet.

2. Vorrichtung (1) nach Anspruch 1, **dadurch gekennzeichnet, dass** die Verarbeitungseinheit (9) konfiguriert ist, um die Bewegung des Teils der Vorrichtung (2) zu deaktivieren, wenn sie eine plötzliche Veränderung des gemessenen biometrischen Werts von dem biometrischen Referenzwert auf den veränderten biometrischen Wert erfasst.

3. Vorrichtung (1) nach einem der vorhergehenden Ansprüche, **dadurch gekennzeichnet, dass** der veränderte biometrische Wert größer als der gemessene biometrische Wert ist.

4. Vorrichtung (1) nach einem der vorhergehenden Ansprüche, **dadurch gekennzeichnet, dass** das Verwaltungssystem (3) bei Verwendung mit einem Steuersystem des Teils in Bewegung zur Verwaltung des Teils in Bewegung verbunden ist.

5. Vorrichtung (1) nach einem der Ansprüche 1 bis 3, **dadurch gekennzeichnet, dass** das Verwaltungssystem (3) bei Verwendung zwischen einer elektrischen Energiequelle (4) und der elektrischen Versorgung der Vorrichtung (2) angeordnet und damit verbunden ist.

6. Vorrichtung (1) nach einem der vorhergehenden Ansprüche, **dadurch gekennzeichnet, dass** der biometrische Referenzwert im Wesentlichen dem zuletzt gemessenen biometrischen Wert gleich ist.

7. Vorrichtung (1) nach einem der Ansprüche 1 bis 5, **dadurch gekennzeichnet, dass** der biometrische Referenzwert im Wesentlichen dem Durchschnitt der zuletzt gemessenen biometrischen Werte gleich ist.

8. Vorrichtung (1) nach einem der vorhergehenden Ansprüche, **dadurch gekennzeichnet, dass** die Verarbeitungseinheit (9) in dem Verwaltungsgerät (3) untergebracht ist.

9. Vorrichtung (1) nach einem der Ansprüche 1 bis 7, **dadurch gekennzeichnet, dass** die Verarbeitungseinheit (9) auf dem Messgerät (7) des biometrischen Werts untergebracht ist.

10. Vorrichtung (1) nach einem der Ansprüche 1 bis 7, **dadurch gekennzeichnet, dass** sie ein Smartphone (12) und/oder ein Tablet und/oder einen Computer umfassend eine eigene Verarbeitungskarte umfasst, wobei die Verarbeitungseinheit (9) durch die Verarbeitungskarte des Smartphones (12) und/oder Tablets und/oder Computers definiert ist.

11. Vorrichtung (1) nach einem der Ansprüche 1 bis 7, **dadurch gekennzeichnet, dass** das Messgerät (7) des biometrischen Werts ein am Arm eines Benutzers tragbares Armband und einen Sensor (14) zum Messen des biometrischen Werts durch Kontakt mit dem Körper des Benutzers umfasst.

12. Vorrichtung (1) nach einem der vorhergehenden Ansprüche, **dadurch gekennzeichnet, dass** das Gerät (7) zum Messen des biometrischen Werts einen Sensor (14) zum Messen des Herzschlags umfasst, sodass der Herzschlag den biometrischen Wert definiert, wobei der biometrische Referenzwert durch einen Herzschlagwert in einem normalen Lebenszustand des Benutzers definiert wird, wobei der veränderte biometrische Wert durch einen Herzschlagwert definiert wird, der größer als der Herzschlagwert des normalen Lebenszustands ist.

13. Vorrichtung (1) nach einem der vorhergehenden Ansprüche, **dadurch gekennzeichnet, dass** das Deaktivierungsignal (11) von der Verarbeitungseinheit (9) an das Verwaltungsgerät (3) drahtlos übertragen wird.

14. Vorrichtung (1) nach einem der vorhergehenden Ansprüche, **dadurch gekennzeichnet, dass** das Messsignal (10) von der Verarbeitungseinheit (7) an das Verarbeitungsgerät (9) drahtlos übertragen wird.

15. Verfahren zum Schutz von Benutzern während der Verwendung einer Vorrichtung (2) mit mindestens einem Teil in Bewegung, umfassend die folgenden Arbeitsschritte:
- Verbinden eines Verwaltungsgeräts (3) des Teils in Bewegung der Vorrichtung (2) mit der Vorrichtung (2), wobei das Verwaltungsgerät (3) zwischen einem aktivierten Zustand, in dem die Bewegung des Teils der Vorrichtung (2) aktiviert ist, und einem Unterbrechungszustand, in dem die Bewegung des Teils der Vorrichtung (2) deaktiviert ist, konfigurierbar ist;
- Messen eines biometrischen Werts des Körpers des Benutzers;
**dadurch gekennzeichnet, dass** es die folgenden Betriebsschritte umfasst:
Vergleichen des gemessenen biometrischen Werts mit einem biometrischen Referenzwert, wobei der biometrische Referenzwert durch einen Anwesenheitszustand des Benutzers in einem normalen Lebenszustand dargestellt wird;
- Senden eines Deaktivierungssignals (11) an das Verwaltungsgerät (3), um das Verwaltungsgerät von dem aktivierten Zustand in den Unterbrechungszustand zu konfigurieren, wenn der gemessene biometrische Wert einen veränderten biometrischen Wert erreicht oder überschreitet, der einen Anwesenheitszustand des Benutzers in einem veränderten Lebenszustand darstellt, um die Bewegung des Teils der Vorrichtung (2) zu deaktivieren, wobei der veränderte biometrische Wert sich vom biometrischen Referenzwert unterscheidet.

## Revendications

1. Appareil (1) pour protéger des utilisateurs pendant l'utilisation d'un appareil (2) ayant au moins une partie en mouvement, comprenant :
- un dispositif de gestion (3) d'un mouvement de la partie de l'appareil (2) en fonctionnement relié à l'appareil (2) ; ledit dispositif de gestion (3) pouvant être configuré entre une condition d'activation, pendant laquelle le mouvement de la partie de l'appareil (2) est activé, et une condition d'interruption, pendant laquelle le mouvement de la partie de l'appareil (2) est désactivé ;
- un dispositif de mesure (7) d'au moins une valeur biométrique associé en fonctionnement au corps de l'utilisateur pour mesurer la valeur biométrique ; **caractérisé en ce qu'**il comprend une unité de traitement (9) fonctionnellement reliée au dispositif de gestion (3) du mouvement et au dispositif de mesure (7) ; l'unité de traitement (9) étant configurée pour :
- recevoir un signal de mesure (10) de la valeur biométrique contenant la valeur biométrique mesurée par le dispositif de mesure (7) ;
- comparer la valeur biométrique mesurée à une valeur biométrique de référence, dans lequel la valeur biométrique de référence est représentée par une condition de présence de l'utilisateur dans une condition de la vie de tous les jours ;
- envoyer un signal de désactivation (11) au dispositif de gestion (3) du mouvement de sorte à configurer le dispositif de gestion de la condition d'activation à la condition d'interruption lorsque la valeur biométrique mesurée et contenue dans le signal de mesure (10) atteint ou dépasse une valeur biométrique modifiée représentant une condition de présence de l'utilisateur dans une condition de vie modifiée de sorte à désactiver le mouvement de la partie de l'appareil (2) ; la valeur biométrique modifiée étant différente de la valeur biométrique de référence.

2. Appareil (1) selon la revendication 1, **caractérisé en ce que** l'unité de traitement (9) est configurée pour désactiver le mouvement de la partie de l'appareil (2) lorsqu'elle détecte une variation soudaine de la valeur biométrique mesurée à partir de la valeur biométrique de référence à la valeur biométrique modifiée.

3. Appareil (1) selon l'une quelconque des revendications précédentes, **caractérisé en ce que** la valeur biométrique modifiée est supérieure à la valeur biométrique mesurée.

4. Appareil (1) selon l'une quelconque des revendications précédentes, **caractérisé en ce que** le système de gestion (3) est relié, en fonctionnement, à un système de commande de la partie en mouvement pour gérer la partie en mouvement.

5. Appareil (1) selon l'une quelconque des revendications de 1 à 3, **caractérisé en ce que** le système de gestion (3), en fonctionnement, est interposé entre et relié à une source (4) d'énergie électrique et à l'alimentation électrique de l'appareil (2).

6. Appareil (1) selon l'une quelconque des revendications précédentes, **caractérisé en ce que** la valeur biométrique de référence est substantiellement égale à la valeur biométrique la plus récemment mesurée.

7. Appareil (1) selon l'une quelconque des revendications de 1 à 5, **caractérisé en ce que** la valeur biométrique de référence est substantiellement égale à la moyenne des valeurs biométriques les plus récemment mesurées.

8. Appareil (1) selon l'une quelconque des revendications précédentes, **caractérisé en ce que** l'unité de traitement (9) est logée sur le dispositif de gestion (3).

9. Appareil (1) selon l'une quelconque des revendications de 1 à 7, **caractérisé en ce que** l'unité de traitement (9) est logée sur le dispositif de mesure (7) de la valeur biométrique.

10. Appareil (1) selon l'une quelconque des revendications de 1 à 7, **caractérisé en ce qu'**il comprend un smartphone (12) et/ou une tablette et/ou un ordinateur comprenant sa/leur propre carte de traitement ; l'unité de traitement (9) étant définie par la carte de traitement du smartphone (12) et/ou de la tablette et/ou de l'ordinateur.

11. Appareil (1) selon l'une quelconque des revendications de 1 à 7, **caractérisé en ce que** le dispositif de mesure (7) de la valeur biométrique comprend un bracelet pouvant être porté sur un bras de l'utilisateur et un capteur (14) pour mesurer la valeur biométrique par contact avec le corps de l'utilisateur.

12. Appareil (1) selon l'une quelconque des revendications précédentes, **caractérisé en ce que** le dispositif (7) pour mesurer la valeur biométrique comprend un capteur (14) pour mesurer la fréquence cardiaque de manière à ce que la fréquence cardiaque définisse la valeur biométrique ; la valeur biométrique de référence étant définie par une valeur de fréquence cardiaque dans une condition de vie normale de l'utilisateur, la valeur biométrique modifiée étant définie par une valeur de fréquence cardiaque supérieure à la valeur de fréquence cardiaque de la condition de vie normale.

13. Appareil (1) selon l'une quelconque des revendications précédentes, **caractérisé en ce que** le signal de désactivation (11) est transmis par l'unité de traitement (9) au dispositif de gestion (3) via une connexion sans fil.

14. Appareil (1) selon l'une quelconque des revendications précédentes, **caractérisé en ce que** le signal de mesure (10) est transmis par l'unité de traitement (7) au dispositif de traitement (9) via une connexion sans fil.

15. Procédé pour protéger des utilisateurs pendant l'utilisation d'un appareil (2) ayant au moins une partie en mouvement, comprenant les étapes de fonctionnement suivantes :
- connecter un dispositif de gestion (3) de la partie en mouvement de l'appareil (2) à l'appareil (2), dans lequel le dispositif de gestion (3) peut être configuré entre une condition d'activation, pendant laquelle le mouvement de la partie de l'appareil (2) est activé, et une condition d'interruption, pendant laquelle le mouvement de la partie de l'appareil (2) est désactivé ;
- mesurer une valeur biométrique du corps de l'utilisateur ;
**caractérisé en ce qu'**il comprend les étapes de fonctionnement suivantes :
comparer la valeur biométrique mesurée à une valeur biométrique de référence, dans lequel la valeur biométrique de référence est représentée par une condition de présence de l'utilisateur dans une condition de la vie de tous les jours ;
- envoyer un signal de désactivation (11) au dispositif de gestion (3) de sorte à configurer le dispositif de gestion de la condition d'activation à la condition d'interruption lorsque la valeur biométrique mesurée atteint ou dépasse une valeur biométrique modifiée représentant une condition de présence de l'utilisateur dans une condition de vie modifiée de sorte à désactiver le mouvement de la partie de l'appareil (2) ; la valeur biométrique modifiée étant différente de la valeur biométrique de référence.
